# EUROPEAN PATENT SPECIFICATION

(11) **EP 3 801 657 B1**
(45) Date of publication and mention of the grant of the patent: **04.09.2024**
(21) Application number: 19701596.9
(22) Date of filing: 18.01.2019
(51) Int. Cl.: A61L 27/22

(54) **METHODS OF PRODUCTION OF FIBROUS FIBRINOGEN SCAFFOLDS AND PRODUCTS THEREOF**
VERFAHREN ZUR HERSTELLUNG VON FASERIGEN FIBRINOGENGERÜSTEN UND DARAUS HERGESTELLTE PRODUKTE
PROCÉDÉS DE PRODUCTION D'ÉCHAFAUDAGES À BASE DE FIBRINOGÈNE FIBREUX ET PRODUITS ASSOCIÉS

(30) Priority: 28.05.2018 DE 102018112678
(43) Date of publication of application: 14.04.2021
(73) Proprietor: Universität Bremen, 28359 Bremen (DE)
(72) Inventor: BRÜGGEMANN, Dorothea, 28201 Brermen (DE); STAPELFELDT, Karsten, 28359 Bremen (DE); MEDNIKOVA, Polina, 76133 Karlsruhe (DE)
(74) Representative: Erbacher, Martin
(86) International application number: PCT/EP2019/051242
(87) International publication number: WO 2019/228676

(56) References cited:
- SEONG SOO A. AN ET AL: "Fibrinogen and fibrin based micro and nano scaffolds incorporated with drugs, proteins, cells and genes for therapeutic biomedical applications", INTERNATIONAL JOURNAL OF NANOMEDICINE, vol. 8, no. 1, 5 September 2013 (2013-09-05), New Zealand, pages 3641 - 3662, XP055225917, ISSN: 1178-2013, DOI: 10.2147/IJN.S43945
- ZHANG LIUDI ET AL: "The influence of surface chemistry on adsorbed fibrinogen conformation, orientation, fiber formation and platelet adhesion", ACTA BIOMATERIALIA, ELSEVIER, AMSTERDAM, NL, vol. 54, 2 March 2017 (2017-03-02), pages 164 - 174, XP029989896, ISSN: 1742-7061, DOI: 10.1016/J.ACTBIO.2017.03.002

## Description

The present invention relates to methods for producing fibrous fibrinogen biomaterials that can be used as three-dimensional scaffolds. The methods of this invention enable the controlled detachment of fibrous fibrinogen scaffolds in vitro. The fibrous fibrinogen biomaterials generated by the methods of this invention can be detached in a solution. Alternatively, the fibrous fibrinogen scaffolds of this invention can be immobilized on a surface. The fibrous fibrinogen biomaterial can be used in medicine, such as in wound healing, regenerative medicine, dermal reconstruction, skin repair, bone vessel repair, blood vessel regeneration, tissue engineering, and implant coatings. The biomaterials can be generated "on-demand" and can be transferred to a site of injury, such as to a wound.

### Background of the invention

Fibrinogen, also referred to as 'Factor I', is a glycoprotein that circulates in the blood of vertebrates. During tissue and vascular injury, it is converted enzymatically by thrombin to fibrin and subsequently to a fibrin-based blood clot. Fibrinogen functions primarily to occlude blood vessels and thereby stop excessive bleeding. However, fibrinogen binds and reduces the activity of thrombin. This activity, sometimes referred to as antithrombin I, is important to limit blood clotting. Accordingly, loss or reduction in antithrombin 1 activity due to mutations in fibrinogen genes or hypo-fibrinogen conditions can lead to excessive blood clotting and thrombosis. Fibrin also mediates blood platelet and endothelial cell adhesion, cell spreading, tissue fibroblast proliferation, capillary tube formation, and angiogenesis. Importantly, fibrin functions to promote tissue revascularization, wound healing, and tissue repair.

Reduced and/or dysfunctional fibrinogens occur in various congenital and acquired human fibrinogen-related disorders. These disorders represent a clinically important group of rare conditions in which individuals may present with severe episodes of pathological bleeding and thrombosis; these conditions are treated by supplementing blood fibrinogen levels and inhibiting blood clotting, respectively. Some of these disorders may also be the cause of diseases, such as liver and kidney diseases.

Fibrinogen is a "positive" acute-phase protein, i.e. its blood levels rise in response to systemic inflammation, tissue injury, and certain other events. It is also elevated in various cancers. Elevated levels of fibrinogen in inflammation as well as cancer and other conditions have been suggested to be the cause of thrombosis and vascular injury that accompanies these conditions.

Tissue engineering is the use of a combination of cells, engineering and materials methods, and suitable biochemical and physicochemical factors to improve or replace biological tissues. Tissue engineering is defined as the "generation of tissue in a test tube" and usually involves the use of a tissue scaffold for the formation of new viable tissue for a medical purpose. Fibrinogen has become highly attractive for tissue engineering scaffolds since it is a naturally occurring blood protein, which contains important binding sites to facilitate cell adhesion.

While most definitions of tissue engineering cover a broad range of applications, in practice the term is closely associated with applications that repair or replace portions of or whole tissues (i.e., bone, cartilage, blood vessels, bladder, skin, muscle etc.). Often, the tissues involved require certain mechanical and structural properties for proper functioning. The term has also been applied to efforts to perform specific biochemical functions using cells within an artificially-created support system (e.g. an artificial pancreas, or a bio-artificial liver). The term regenerative medicine is often used synonymously with tissue engineering, although those involved in regenerative medicine place more emphasis on the use of stem cells or progenitor cells to produce tissues.

In wound healing and tissue engineering applications, a wide range of proteins are used, which are naturally found in the human body. The protein fibrinogen is of particular interest for these applications as it is involved in blood coagulation and blood clot formation, thus leading to the formation of new tissue after an injury. Fibrous protein scaffolds are particularly important for wound healing applications since cells are naturally embedded into a fibrous network of extracellular matrix proteins. Therefore, a wide range of fibrous protein scaffolds is currently used as biomaterials in regenerative medicine. The fibrous protein scaffolds determine the shape that will subsequently be populated by cells.

Fibrillogenesis of fibrinogen is the process of producing fibrinogen fibers from fibrinogen. However, fibrillogenesis of fibrinogen by previously reported methods have only led to fiber formation with a very low yield and a low surface coverage on substrates. One of the described methods to produce fibrinogen fibers in vitro is the method of electrospinning. This method has facilitated a wide range of cell culture studies on electrospun fibrinogen nanofibers. The process of electrospinning usually involves large concentrations of fibrinogens, high electric fields with voltages of more than 20 kV as well as organic solvents, such as 1,1,1,3,3,3-hexafluoro-2-propanol.

US20150297791 A1 discloses a method for the production of fibrous scaffolds using different polymers and the method of electrospinning. Subsequently, proteins like fibrinogen can be added to the scaffold, and thereby colonize the scaffold. Consequently, this method produces a mixture of polymers and extracellular matrix proteins like fibrinogen.

EP2844307 B1 discloses a method for the production of an extracellular matrix which can be used for wound healing and tissue regeneration. The composition that is produced with this method comprises a large number of ingredients, such as coagulating agents, bulking agents and foaming agents. One embodiment also relates to the addition of fibrinogen. However, when fibrinogen is used in the method as described in EP2844307 B1, fibrinogen will be converted enzymatically into fibrin. Accordingly, no scaffold comprising fibrinogen can be generated using the method of EP2844307 B1.

Importantly, fibrous fibrinogen scaffolds are of outmost interest for biomedical applications, such as for wound healing and tissue engineering applications. The reason for this is that fibrinogen is one of the key factors in blood coagulation and blood clot formation, which leads to wound healing via different stages. So far, a diverse range of fibrinogen and fibrin scaffolds, such as microspheres, microbeads, microfibers, nanoparticles, nanofibers, and hydrogels have been prepared for versatile biomedical applications.

Previous approaches to prepare fibers from fibrinogen and fibrinogen scaffolds have different drawbacks or restrictions. In previous studies, hydrogels from fibrin have been used as cell culture substrates in experimental studies. Fibrin is obtained when fibrinogen is incubated with the enzyme thrombin, which cleaves off the fibrinopeptides A and B, thus resulting in the molecule fibrin. Therefore, fibrin hydrogels do not contain the native form of fibrinogen due the addition of the enzyme thrombin during the preparation.

Methods have also been disclosed that enable the preparation of free-standing, three-dimensional hydrogels from fibrinogen. However, these methods require the addition of other polymers like hyaluron or polyethylene glycol or modifications of the fibrinogen molecule itself to facilitate a stable hydrogel preparation. Moreover, methods to prepare fibrin hydrogels require the addition of the enzyme thrombin, which cleaves off the fibrinopeptides A and B from the original fibrinogen molecule, thus inducing a substantial change in the molecular structure. Importantly, the use of the enzyme thrombin makes the preparation of fibrin hydrogels a very expensive method.

Fibrillogenesis of fibrinogen in *vitro* has so-far only been described under non-physiological buffer conditions, i.e. acidic pH, oxidative agents, or in the presence of solvents like ethanol. These can interfere with the native protein conformation and result in reduced bioactivity of fibrinogen. Additionally, buffer-induced fibrillogenesis methods have so-far only achieved to produce fibrous scaffolds with a low fiber yield. Moreover, some studies described the fibrillogenesis of fibrinogen on hydrophobic surfaces. However, using these methods, fibrinogen fiber yield was very low and these fibers were only sparsely distributed on the underlying substrate and no dense fibrous scaffold suitable for tissue engineering could be obtained. Due to low fiber density, no three-dimensional fibrous scaffolds were obtained. Besides, hydrophobic substrates, which can induce fibrinogen fibrillogenesis, can lead to undesired side effects in cell culture systems and can result in (partial) protein denaturation upon adsorption.

Further methods make use of electrospinning of fibrinogen to produce fibers in *vitro.* Seong Soo A. An et al., International Journal of Nanomedicine, vol. 8, no. 1, 3641-3662 discloses tissue restoration with fibrinogen (Fbg) micro- and nanoscaffolds. The scaffold can be made of Fbg micro/nanofibers. The nanofibrous scaffolds is fabricated by electrospinning. To stabilize the shape of the scaffolds, they can be crosslinked, e.g. by formaldehyde or glutaraldehyde. The scaffold can be used e.g. in wound healing. I

However, the use of high electric fields in combination with organic solvents, such as 1,1,1,3,3,3-hexafluoro-2-propanol in electrospinning can negatively influence the bioactivity of electrospun fibrinogen scaffolds. Besides, the use of high voltage equipment needed for electrospinning requires high concentrations of fibrinogen in the range of more than 100 mg/ml. Both these factors make electrospinning a very expensive method. Additionally, during electrospinning, fibrinogen nanofibers are often collected on an external moveable collector in order to prepare macroscopic scaffolds. This fiber collection adds another step into the electrospinning procedure, which makes the electrospinning of fibrous fibrinogen scaffolds more complex and more expensive.

The above-mentioned fabrication methods to obtain fibrous fibrinogen scaffolds or hydrogels from fibrinogen have been used with different parameters and variations. However, so far none of the previous approaches has overcome the limitations and disadvantages stated above. Importantly, no controlled detachment of fibrinogen scaffolds in vitro has previously been reported. To overcome such problems stated above, novel strategies to prepare large amounts of fibrous fibrinogen scaffolds with a high yield of fibers demonstrating a high surface coverage in a cheap, and reproducible way are urgently needed.

The underlying task of this invention is to develop fibrous fibrinogen patches similar to the fibrous fibrinogen structures that are located in the provisional Extracellular Matrix (ECM), which is involved in wound healing in nature. Therefore, the aim of this invention is to establish a simple and reproducible routine to prepare three-dimensional nano- and microfibrous scaffolds from fibrinogen, which can either be detached "on demand" to obtain free-standing protein patches or which are selectively immobilized on a substrate. With this controlled release from the underlying substrate, it is possible to obtain free-floating fibrous protein patches, which can be transferred to injured tissue areas which facilitates versatile applications in regenerative medicine. During this process, the scaffold composition and morphology, the hierarchical fiber assembly and the scaffold dimensions as well as their mechanical properties can be tailored to create universal fibrous fibrinogen scaffolds as novel biomaterials for different areas of regenerative medicine, e.g. skin and bone repair, nerve regeneration or blood vessel regeneration.

It is a further aim of this invention to provide a simple and well-controllable method to prepare fibrous fibrinogen scaffolds, which only requires cost-efficient lab equipment and consumables. The composition, morphology and hierarchical fiber assembly achieved by this invention shall be reproducibly controlled to facilitate the preparation of fibrous fibrinogen scaffolds with high fiber yield, coverage and scaffold dimensions in the centimeter range.

The problem solved by this invention is to develop a method to generate fibrous fibrinogen biomaterials composed of nano- and/or microfibrous fibrinogen scaffolds, which can either be detached "on demand" to obtain free-standing protein patches, or which are selectively immobilized on a substrate. The problem is solved by providing a method that enables a controlled self-assembly of fibrinogen fibers by fibrillogenesis in solution or solubilized on a substrate *in vitro.* The methods of this invention are successful in generating a fibrous fibrinogen biomaterial in an enzyme- and additive-free buffer system at neutral pH. Accordingly, the methods of this invention facilitate the preparation of micro- or nanofibrous fibrinogen scaffolds, which appear to be identical to those fibrinogen fibers seen in *vivo* due to the physiological conditions used.

The above problem is solved in a first aspect by providing a method of generating a fibrous fibrinogen biomaterial, comprising
a) Providing a substrate,
b) Optionally, cleaning said substrate,
c) Optionally, modifying of at least one surface of said substrate,
d) Adding a fibrinogen solution to said substrate, or submerging said substrate in said fibrinogen solution,
e) Adding a salt buffer and/or an aqueous buffer and/or water to said substrate, or submerging said substrate in said salt buffer and/or said aqueous buffer and/or said water,
f) Optionally drying said substrate,
g) Optionally, adding a salt buffer and/or an aqueous buffer and/or water to said substrate, or submerging said substrate in said salt buffer and/or said aqueous buffer and/or said water,
h) Optionally, drying said substrate,
i) Optionally, performing one or more repetitions of steps g) to h),
j) Fixating said substrate, and
k) Washing said substrate,
thereby generating said fibrous fibrinogen biomaterial.

The above method is useful in the preparation of immobilized nanofibrous fibrinogen scaffolds using salt-induced self-assembly. The method is further useful in the preparation of fibrinogen microfibers by rehydration. However, for the preparation of fibrinogen microfibers by rehydration, a further step of drying said substrate is preferably performed after adding said fibrinogen solution to said substrat in step d) and prior to adding said salt buffer in step e).

In one preferred embodiment, the fibrous fibrinogen biomaterial that is generated using the method of this invention is composed of three-dimensional fibrinogen fibers.

The term "fibre" or "fibers" as used herein shall refer to any slender, threadlike structure assembled by protein components. Preferably, the term "fiber" or "fibers" shall mean filamentous structures such as fibrigenous or collagenous elastic connective tissue fibers. Importantly, the basic components of connective tissue are cells and extracellular protein fibers embedded in a matrix or ground substance of large carbohydrate molecules and carbohydrate-protein complexes called mucopolysaccharides. "Fibrous" in the context of this invention shall refer to any structure consisting of, containing, or resembling fibers. A structure can further be defined as being "fibrous", if the structure can be separated into individual fibers.

Further preferred is that the fibrous fibrinogen biomaterial that is generated using the method of this invention can be used as a scaffold. A "scaffold" in the context of this invention shall be any support system or framework for the formation of new viable tissue. Such a scaffold can be colonized by cells to generate new tissue. Such engineered tissue can subsequently improve or replace biological tissues, such as damaged tissue. Accordingly, the scaffold as generated using the methods of this invention can be used for medical purposes and cosmetic purposes.

In further preferred embodiments of the present invention, the fibrinogen fibers generated by the methods of this invention have a diameter of between 0.1 nm and 10000 nm, preferably between 1 nm and 1000 nm, more preferably between 10 nm and 500 nm, and most preferably between 100 nm and 200 nm.

Further preferred is that the fibrous fibrinogen biomaterial to be generated by the methods of this invention has a hydrodynamic radius of at least 0.00001 mm, preferably of at least 0.001 mm, more preferably of at least 0.01 mm, further preferably of at least 0.1 mm, even more preferably of at least 1 mm, and most preferably of at least 10 mm.

In a preferred embodiment, the substrate as provided in step a) of the method of this invention is composed of a solid material, such as a solid glass material. The substrate can optionally comprise at least one surface composed of a metal selected from gold, silver, copper, gold-platted copper, tinned copper, aluminum, platinum, indium, tungsten, beryllium, gallium, lithium, calcium, magnesium, zinc, titanium, zirconium, hafnium, and mixtures thereof. Further preferred is that the metal surface is composed of gold. Alternatively, the substrate comprises at least one surface composed of a polymer, such as polylactide (PLA), polystyrene, polybutyrate adipate terephthalate (PBAT), or mixtures thereof, preferably wherein said polymer is polylactide (PLA). The substrate can comprise at least one surface composed of a metal and one surface composed of a polymer matrix, or comprise at least one surface composed of a metal only, or comprise at least one surface composed of a polymer matrix only.

Addtionally preferred is that the cleaning step b) is used to remove grease, dirt, and/or organic residues from the substrate. The substrate can be cleaned with Piranha solution, hexanes, potassium dichromate (K₂Cr₂O₇), and/or acetone. Alternatively a potassium hydroxide in an alcohol solution, such as a 1-10% KOH in 1:1 isopropropyl alkohol - water solution or a sodium hydroxide in an alcohol solution, such as a 1-10% NaOH in 1:1 isopropropyl alcohol solution can be used for cleaning the substrate. A further option for cleaning the substrate in step b) is the use of a potassium hydroxide/sodium hydroxide and potassium phosphate solution, such as a solution containing 1% KOH and 0.25% K₃PO₄ in water. Preferably, the substrate in step b) is cleaned using piranha solution. "Piranha solution", in the context of this invention, shall be a mixture of sulfuric acid and hydrogen peroxide. One example is a solution composed to 3 parts of sulfuric acid (H₂SO₄) and to 1 part of hydrogen peroxide (H₂O₂). The concentration of hydrogen peroxide as used can be any concentration between 1 and 70%, preferably between 5 and 60%, more preferably between 10 and 50%, even more preferably between 20 and 40%, and most preferably around 30%. Such a Piranha solution can effectively remove organic residues from substrates. When step b) of this method is performed using Piranha solution, it is further advantageous to subsequently dry the substrate in Nitrogen (N₂). Alternatively, plasma cleaning can be used in step b) to remove all organic matter from the surfaces of the substrate. The person skilled in the art is well aware of how to perform plasma cleaning. For example, the substrate can be cleaned by exposing the substrate to an ionized gas, i.e. to plasma. This method is generally performed in a vacuum chamber utilizing oxygen and/or argon gas. To any of the solutions as described above, a laundry detergent can be added.

In a further preferred embodiment, the modifying step c) of the method of this invention is selected from silanization, esterification, phosphorization, hydrosilyation, physisorption, aldehyde modification, carboxylate modification, amine modification, epoxy modification, and mixtures thereof. Further preferred is that the modification is silanization.

Another preferred embodiment relates to the silanization of the substrate, which is preferably performed by immersing the substrate in a silanization agent selected from 3-aminopropyltriethoxysilane (APTES), (3-aminopropyl)-dimethyl-ethoxysilane (APDMES), N-(2-aminoethyl)-3-aminopropyltrimethoxysilane (AEAPS), 3-aldehydepropyltrimethoxysilane (APMS), mercaptopropyltrimethoxysilane (MPTMS), mercaptopropyltriethoxysilane (MPTES), biotin 4-nitrophenyl ester (BNPE), 11-hydroxyundecyl-phosphonate (HUP), organopolysiloxane, trimethylchlorosilane, and mixtures thereof, preferably wherein said silanization agent is 3-aminopropyltriethoxysilane (APTES).

Step c) of the method of this invention can be performed on at least one surface of the substrate, i.e. it can be performed on one, two, three, four, or any number of surfaces of the substrate. If step c) is performed on one surface of the substrate, the fibrinogen solution added to said substrate in step d) should preferably be added to the surface of the substrate that has been modified in step c). It is further preferred that if the substrate comprises only one surface composed of a metal selected from gold, silver, copper, gold-platted copper, tinned copper, aluminum, platinum, indium, tungsten, beryllium, gallium, lithium, calcium, magnesium, zinc, titanium, zirconium, hafnium, and mixtures thereof, preferably wherein the metal surface is composed of gold, the fibrinogen solution added to said substrate in step d) should preferably be added to the surface of the substrate that is composed of the metal, such as of gold. Alternatively, it is also preferred that if the substrate comprises one surface composed of a polymer, this polymer is preferably polylactide (PLA).

In a further preferred embodiment, the fibrinogen solution as added in step d) of the method of this invention has a fibrinogen concentration of between 0.01 and 1000 mg/ml, preferably between 0.1 and 100 mg/ml, more preferably between 0.5 and 50 mg/ml, and most preferably between 1 and 5 mg/ml. Accordingly, low fibrinogen concentrations are sufficient (1 to 5 mg/ml) for the method of this invention, compared to high concentrations needed for electrospinning methods, where the protein concentration of fibrinogen needs to be around 100 to 200 mg/ml.

Another preferred embodiment relates to the fibrinogen solution as added in step d) of the method of this invention, wherein said fibrinogen solution is an aqueous fibrinogen solution.

Further preferred is that if a salt buffer is added in step e) and optionally also in step g) of the method of this invention, said salt buffer as added preferably comprises at least one component selected from sodium phosphate, sodium chloride, ammonium carbonate, ammonium phosphate, boric acid, citric acid, lactic acid, phosphoric acid, potassium chloride, potassium citrate, potassium metaphosphate, potassium phosphate (monobasic), sodium acetate, sodium citrate, sodium lactate, sodium phosphate (dibasic), sodium phosphate (monobasic), and mixtures thereof. It is further preferred that if the salt of the buffer as added in step e) is sodium phosphate, the concentration of sodium phosphate should be at least 1 mM, preferably at least 5 mM, more preferably at least 10 mM, even more preferably at least 25 mM, and most preferably at least 50 mM. Alternatively, if the salt of the buffer as added in step e) is not sodium phosphate, the concentration of the salt needed might be higher, such as at least 10 mM, more preferably at least 50 mM, even more preferably at least 100 mM, and most preferably at least 1M.

Further preferred is that the salt buffer as added is phosphate-buffer-saline (PBS), optionally wherein said PBS comprises sodium chloride, potassium chloride, sodium phosphate, and potassium phosphate. The fiber preparation methods of this invention preferably involve physiological buffers at neutral pH. Accordingly, a solution used in this method has a pH of between 1 and 14, preferably between 4 and 12, more preferably between 6 and 10, even more preferably between 7 and 9, and most preferably between 7.7 and 8.3. Importantly, the physiological buffer conditions used in this invention without the need for solvents or a low pH or other denaturing agents enable fiber preparation of a fibrous fibrinogen biomaterial that maintains the biological functionality of fibrinogen.

Another preferred embodiment then relates to the method of this invention, wherein fibrous fibrinogen nanofibers are generated when said optional steps g), h), and i) of said method are not performed, preferably wherein said buffer used in step e) is a salt buffer. "Nanofibers" in the context of this invention shall refer to fibers in the range of nanometers, i.e. having a diameter of between 0.01 nm and 10000 nm, preferably between 1 nm and 1000 nm, more preferably between 10 nm and 500 nm, and most preferably 100 nm and 300.

A further preferred embodiment then relates to the method of this invention, wherein fibrous fibrinogen microfibers are generated when steps g), h), and i) of said method are performed, preferably wherein said buffer used in step e) and step g) is an aqueous buffer. The use of an aqueous buffer in step e) and step g) enables the re-hydration of adsorbed fibrinogen fibers in said aqueous buffer. More specifically, the use of an aqueous buffer in step e) of the method of this invention enables the rehydration of the planar layers of the fibrous fibrinogen biomaterial as generated. Preferably, multiple washing steps in an aqueous buffer will be performed. This will allow multiple rehydration events, so that preferably the layers of the fibrous fibrinogen biomaterial will multiply. It is a further preferred embodiment of this invention to use multiple repetitions of step e) in an aqueous buffer to generate multiple layers of fibrous fibrinogen biomaterial that will assemble in form of fibrinogen fibers. Preferably, when an aqueous buffer is used in step e) of this method, this step will be repeated at least 2 times, more preferably at least 3 times, even more preferably at least 4 times, or any other number of times, such as at least 5 times, at least 6 times, at least 7 times, at least 8 times, at least 9 times, or at least 10 times. When an aqueous buffer is used in step e) of the method of this invention, the fibrous fibrinogen fibers, which are generated by the method of this invention, are microfibers. The aqueous buffer as used in step e) of the method of this invention generates microfibers by re-hydrating adsorbed fibrinogen layers or fibers in said aqueous buffer.

Drying said substrate in step f) of the method of this invention is preferably performed by incubating the substrate in a chamber. Re-hydration of fibres is performed under "ambient conditions". Salt-induced self-assembly of nanofibers is performed by incubating the substrate in a chamber, wherein said chamber is characterized in having a humidity of less than 50%, more preferred of between 10% and 40%, and most preferred between 20% and 30%. The skilled person is well aware of how to determine and how to adjust the humidity of a chamber. For example, the use of a climatic chamber enables the specific adjustment of humidity and temperature. The temperature that is preferably used in the method of this invention is between 15°C and 25°C, more preferably between 17°C and 23°C, even more preferably between 19°C and 21°C, and most preferably around 20°C. Moreover, the drying of said substrate is also preferably performed at a temperature of between 15°C and 25°C, more preferably between 17°C and 23°C, even more preferably between 19°C and 21°C, and most preferably around 20°C. Importantly, salt-induced self-assembly of nanofibers is largely impossible by incubating the substrate in a chamber, wherein said chamber is characterized in having a humidity of more than 50%.

A further preferred embodiment then relates to the fixating step g) of the method of this invention, which is preferably performed by incubating the substrate in a fixation agent, preferably in the vapor of a fixation agent, wherein said fixation agent is selected from paraformaldehyde, formaldehyde, glutaraldehyde, mercury oxide, lead oxide, osmium oxide, trichloroacetic acid, acetic acid, and mixtures thereof, preferably wherein said fixation agent is paraformaldehyde. Importantly, incubating the substrate in the vapor of a fixation agent instead of a liquid fixation agent is less damaging for the fibrous fibrinogen biomaterial to be generated. Moreover, the use of a liquid phase fixation agent could subsequently initiate the rehydration of fibrinogen fibres. To prevent rehydration of fibrinogen fibres, incubating the substrate in the vapor of the fixation agent has been shown to be advantagous for the method of the present invention. Vapor fixation should preferably occur in a closed box. Further preferred is that the fixation step is at least 2 hours, preferably 12 hours, more preferably at least 24 hours. The concentration of fixation agent used can vary. If paraformaldehyde (PFA) is used as a vapor, the concentration of paraformaldehyde (PFA) is at least 4%, more preferabaly at least 20%, even more preferably at least 30%, and most preferably around 37%. If glutaraldehyde is used as a vapor, the concentration of glutaraldehyde is at least 10%, more preferabaly at least 20%, even more preferably at least 30%, further preferably at least 40%, and most preferably around 50%. Contrary, if liquid PFA is used, the contration of PFA is at least 1%, preferably at least 3%, and most preferably around 4%.

If glutaraldehyde is used as a liquid fixation agent, the concentration of glutaraldehyde is at least 0.5%, preferably at least 1%, most preferably around 2%.

A further embodiment of this invention then relates to the method of this invention, wherein the fixating step g) of the method of this invention is performed by incubating the substrate in the vapor of a fixation agent, such as PFA. This step is then followed by another liquid fixation, for example by incubating the sample in liquid PFA. The duration of the former fixation step should be at least 2 hours, preferably 12 hours, more preferably at least 24 hours. The duration of the latter fixation step should be at least 10 minutes, more preferably at least 20 minutes, and most preferably at least 30 minutes.

Another preferred embodiment then relates to the method of this invention, wherein the washing step k) is performed in an aqueous solution. This aquous solution can, for example, be water. If the washing step k) is performed, said generated fibrous fibrinogen biomaterial is mobile in a solution, optionally wherein said generated mobile fibrous fibrinogen biomaterial can be transferred, such as transferred to an injured tissue. Multiple repetitions of said washing step k) might be advantagous. Preferably, the washing step k) will be repeated at least twice, more preferably at least 3 times.

A further embodiment then relates to the alternative that the washing step k) of the method of this invention is performed and said generated fibrous fibrinogen biomaterial is mobile in a solution., preferably wherein said substrate used to generate mobile fibrous fibrinogen biomaterial is a substrate composed of a glass material. "Mobile in a solution" in the context of this invention shall refer to any free-moving fibrous fibrinogen scaffolds, i.e. scaffolds composed of multiple fibrinogen fibers that are not immobilized on a substrate. Such detachment of fibrous fibrinogen biomaterial enables that said mobile fibrous fibrinogen biomaterial can subsequently be transferred, such as transferred to a side of injury or to an injured tissue.

Another embodiment then relates to the alternative that when said modifying step c) is performed prior to addition of the fibrinogen solution to the substrate in step d), and wherein said modifying is preferably silanization of at least one surface of said substrate, said fibrous fibrinogen biomaterial to be generated will be immobilized on said silanized surface of said substrate.

A further embodiment then relates to the alternative that no modifying step c) is performed prior to addition of the fibrinogen solution to the substrate in step d), and wherein said substrate comprises at least one surface composed of a metal, such as gold, or comprises at least one surface composed of a polymer, such as polylactide (PLA), said fibrous fibrinogen biomaterial to be generated will be immobilized on said silanized surface of said substrate
Accordingly, it is an advantage of the method of the present invention that free-moving scaffolds or immobilized fibrous fibrinogen scaffolds can be prepared just by including or omitting one surface modification step in the procedure, i.e. by tailoring the underlying surface chemistry, and/or by choosing the underlying substrate material. This step is easy to include or exclude, which enables the tailored design of fibrous fibrinogen biomaterial, designed to be suited for the specific purpose as needed.

A further embodiment then relates to the alternative that the washing step k) of the method of this invention is not performed. In absence of this washing step k), said fibrous fibrinogen biomaterial to be generated will be immobilized on the surface of said substrate.

Immobilized fibrous fibrinogen biomaterial will preferably be generated on a substrate when said modifying step c) is performed prior to addition of the fibrinogen solution to the substrate in step d), and wherein said modifying is preferably silanization of at least one surface of said substrate, and additionally a washing step k) of the method of this invention is not performed.

With a tailored combination of substrate modification, fixation and subsequent washing, the scaffolds can either be detached from the underlying substrate to obtain free-floating protein patches in solution or they are systematically immobilized on the substrate. With this versatile process, it is possible to prepare fibrous fibrinogen scaffolds "on demand" for a wide range of biomedical applications. This controlled detachment procedure can also be applied to planar fibrinogen films.

Another aspect of this invention then relates a fibrous fibrinogen biomaterial, produced by the method of this invention. A further aspect of this invention also relates a composition comprising the fibrous fibrinogen biomaterial, produced by the method of this invention.

In another aspect, this invention also relates to the fibrous fibrinogen biomaterial generated by the method of this invention or a composition comprising said fibrous fibrinogen biomaterial for use in tissue engineering, wound healing, regenerative medicine, nerve regeneration, dermal reconstruction, skin and/or bone vessel repair, blood vessel regeneration, implant coatings, biological filters, pharmaceutical screenings, pharmacological screenings, toxicological screenings, and/or biosensors.

"Wound healing" shall be defined as a complex process in which the skin, and the tissues under it, repair themselves after injury. Wound healing has a discrete timeline of physical attributes (phases) constituting the post-trauma repairing process. In undamaged skin, the epidermis (surface layer) and dermis (deeper layer) form a protective barrier against the external environment. When the barrier is broken, a regulated sequence of biochemical events is set into motion to repair the damage. This process is divided into predictable phases: blood clotting (hemostasis), inflammation, tissue growth (proliferation), and tissue remodeling (maturation). Blood clotting may be considered to be part of the inflammation stage instead of a separate stage.

"Regenerative medicine" shall be understood as the aid of regeneration of any tissue or organ. Regenerative medicine can be the process of replacing, engineering or regenerating human cells, tissues or organs to restore or establish normal function. The need for regenerative medicine might be necessary because of an accident or injury, or because a subject, such as a patient, is suffering of a degenerative disease. During a degenerative disease, cells or even entire tissues degenerate. One example of a degenerative disease is a neurodegenerative disease, where commonly neurons or glia cells die to a large extend. The death of cells, in this case neurons or glia cells, then leads to the degeneration of larger areas of the tissues affected, such as certain brain areas.

The fibrous fibrinogen biomaterial can also be used for pharmaceutical screenings.

Tissue engineering based on the fibrous fibrinogen biomaterial of this invention enables the generation of tissue in a test tube. This tissue can subsequently be used to perform pharmaceutical, pharmacological, and/or toxicological screenings in vitro.

The fibrous fibrinogen biomaterial produced according to this invention can further be used in "biological filters" for filtration purposes in the biotechnological field. For example, the fibrous fibrinogen biomarials can further be used in from of a biological filter, such as a "fibrinogen-filter" or a "thrombin-filter" for the filtration of biological fluids, such as of blood. Accordingly, such a "fibrinogen-filter" produced according to this invention could be used to preserve blood by preventing blood coagulation by filtering, e.g., thrombin from the remaining blood components.

Another aspect of this invention then relates to a fibrous fibrinogen biomaterial produced by the method of this invention or a composition comprising said fibrous fibrinogen biomaterial for use as a medicament.

A medical "use" in the context of this invention shall preferably refer to a method for preventing or treating a disease in a subject, wherein the method comprises a step of administering to the subject a therapeutically effective amount of the fibrous fibrinogen biomaterial produced by the method of this invention for use in medicine.

Another aspect of this invention then relates to a fibrous fibrinogen biomaterial produced by the method of this invention or a composition comprising said fibrous fibrinogen biomaterial for use in the treatment of a degenerative disease, a wound, a dermal disease, a vessel damage, and/or skin damage.

The references to the methods of treatment by therapy or surgery or in vivo diagnosis methods in this description are to be interpreted as references to compounds, pharmaceutical compositions and medicaments of the present invention for use in those methods.

A further aspect of this invention then relates to the method of treating a subject suffering from a degenerative disease, a wound, a dermal disease, vessel damage, and/or skin damage by administering to the subject a fibrous fibrinogen biomaterial produced by the method of this invention or a composition comprising said fibrous fibrinogen biomaterial.

In the context of the present invention the term "subject" or "patient" preferably refers to a mammal, such as a mouse, rat, guinea pig, rabbit, cat, dog, monkey, or preferably a human, for example a human patient. The subject of the invention may be at danger of suffering from a disease, or suffer from a disease. A more detailed description of medical indications relevant in the context of the invention is provided herein elsewhere.

Treatment is meant to include, e.g., treating, delaying or alleviating disease progression, reducing the symptoms of, or curing the disease or condition. An "effective amount" is an amount of the fibrous fibrinogen biomaterial produced by the method of this invention or of a composition comprising said fibrous fibrinogen biomaterial as described herein that alleviates symptoms as found for the disease to be treated, such as a degenerative and/or a skin disease. Alleviating is meant to include, e.g., preventing, treating, reducing the symptoms of, or curing the disease or condition. The invention also includes a method for treating a subject at risk for a development and/or progression of a disease, wherein a therapeutically effective amount of the fibrous fibrinogen biomaterial produced by the method of this invention is administered to the patient. Being at risk for the disease can result from, e.g., a family history of the disease, a genotype, which predisposes to the disease, or phenotypic symptoms, which predispose to the disease. In one embodiment, as used herein, the term "prevention" or "preventing" when used in the context of a subject refers to stopping, hindering, and/or slowing down the development or onset of a disease and in particular the symptoms associated with the disease.

Another aspect of this invention then relates to the use of a fibrous fibrinogen biomaterial produced by the method of this invention or a composition comprising said fibrous fibrinogen biomaterial for the manufacture of a medicament for use in the prevention and/or treatment of a degenerative disease, a wound, a dermal disease, a vessel damage, and/or skin damage.

A further aspect of this invention then relates method of inhibiting bleeding at a target site in a patient's body, said method comprising delivering the fibrous fibrinogen biomaterial produced by the method of this invention or a composition comprising said fibrous fibrinogen biomaterial to a target site, a bleeding tissue, an abraded tissue surface, and/or a damaged tissue surface in an amount sufficient to inhibit said bleeding.

"Bleeding", as referred to in this invention, shall be understood as the act, fact, or process of losing blood or having blood flow, i.e. the escape of blood from the circulatory system. Bleeding can also be referred to as haemorrhaging, i.e. blood escaping from the circulatory system.

Another aspect of this invention then relates to a method for delivering a bioactive substance to a target site in a patient's body, said method comprising delivering the fibrous fibrinogen biomaterial produced by the method of this invention in combination with a bioactive substance to the target site.

In a preferred embodiment, the target site is a tissue selected from muscle, skin, epithelial tissue, connective tissue, supporting tissue, nerve tissue, ophthalmic and other sense organ tissue, vascular tissue, cardiac tissue, gastrointestinal organ tissue, pleura and other pulmonary tissue, kidney, endocrine glands, male and female reproductive organs, adipose tissue, liver, pancreas, lymph, cartilage, bone, oral tissue, mucosal tissue, spleen tissue, abdominal organ tissue, and combinations thereof.

Further preferred is that the target site is a bleeding site or bleeding tissue surface of a patient, or an abraded or damaged tissue surface of a patient.

Another preferred embodiment then relates to the target site, which is a void region within the selected tissue. Further preferred is that the void region is selected from tissue divots, tissue tracts, intravertebral spaces, and/or body cavities.

Another preferred embodiment then relates to the target site, which is a tissue surface.

The tissue surface preferably comprises an organ surface selected from the group consisting of a liver surface, a skin surface, a spleen surface, a heart surface, a kidney surface, an intestine surface, a blood vessel surface, a vascular organ surface, and combinations thereof.

A further aspect of this invention then relates to a kit comprising:
a) a composition comprising a fibrous fibrinogen biomaterial produced by the method of this invention,
b) written instructions to apply the fibrous fibrinogen biomaterial onto a target site on a tissue; and
c) optionally, a container holding the composition and the written instructions.

The present invention will now be further described in the following preferred nonlimiting examples with reference to the accompanying figures.

In the figures, the figures show the following as described below.

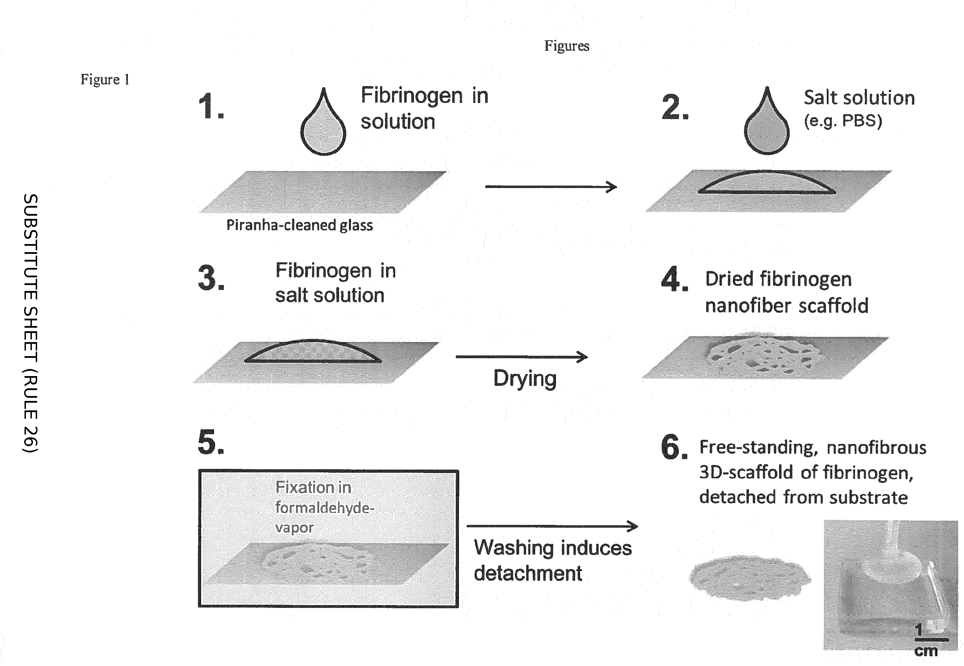

Figure 1: Schematic of the procedure to prepare free-floating nanofibrous fibrinogen scaffolds using salt-induced self-assembly. Piranha-cleaned glasses are used as substrates for fiber formation. Fixation in formaldehyde vapor then leads to controlled scaffold detachment upon subsequent washing.

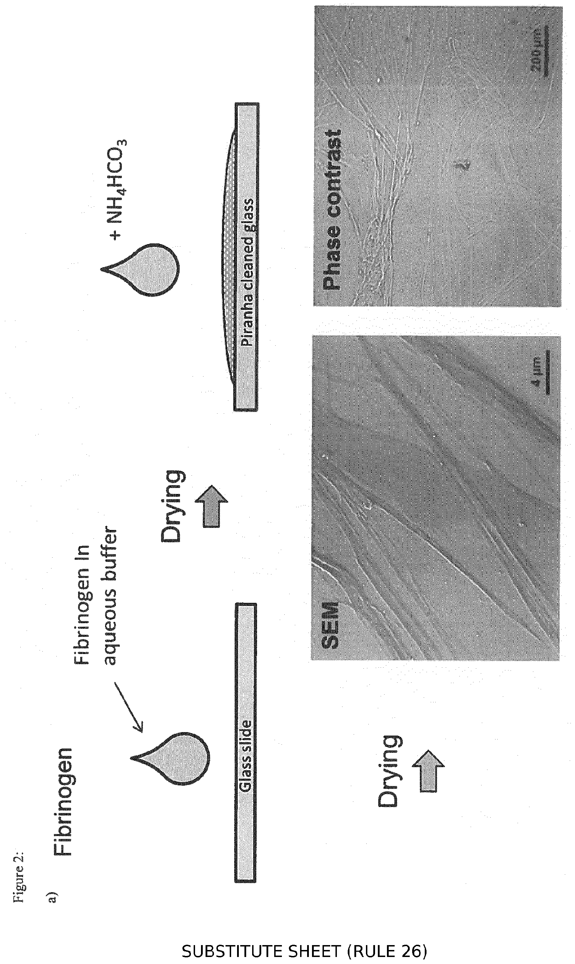

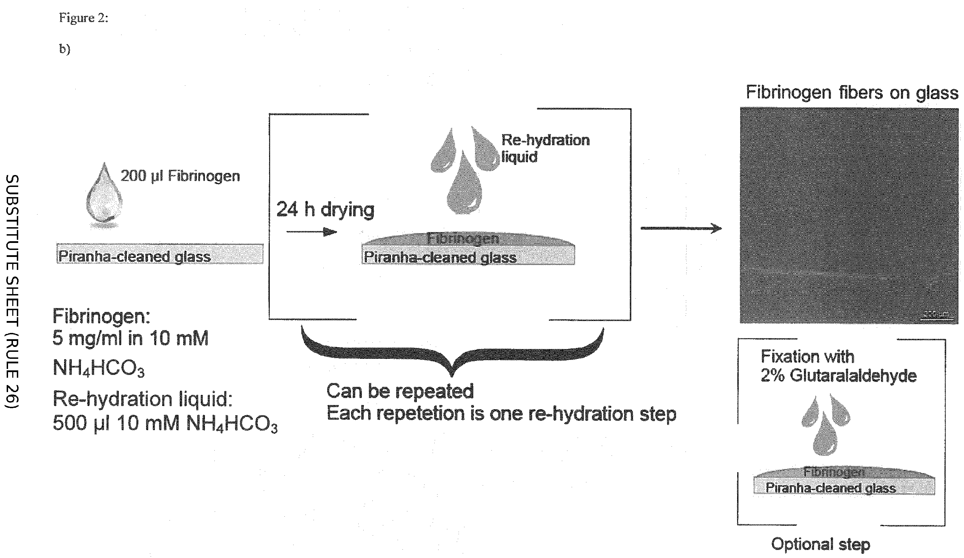

Figure 2: Preparation of fibrinogen microfibers by rehydration. a) Schematic of the procedure to prepare fibrinogen microfibers by rehydration. b) Piranha-cleaned glasses are used as substrates for fiber formation. A 5 mg/ml in 10 mM NH₄CO₃ solution is added to the glass substrate. A 24 h drying step is followed by a rehydration step using 10 mM NH₄CO₃ as a rehydration liquid. The latter rehydration step can be repeated multiple times. Optionally, the fibrinogen fibres that have been generated can be fixated using a fixation agent, such as 2% glutaraldehyde.

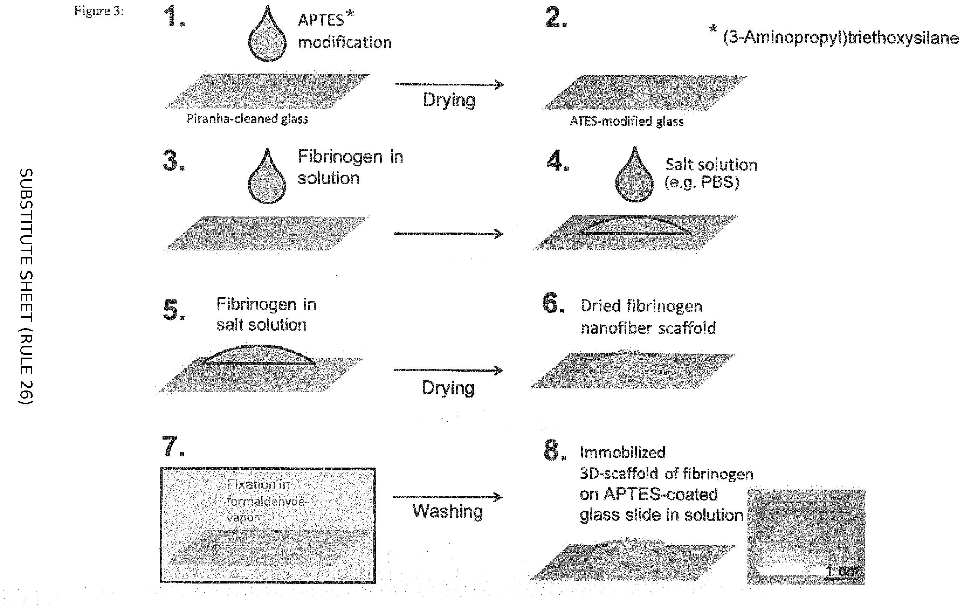

Figure 3: Schematic of the preparation of immobilized nanofibrous fibrinogen scaffolds using salt-induced self-assembly. An APTES-modification is introduced on the glass slide prior to fiber assembly. After fixation in formaldehyde vapor and subsequent washing, the fibrous fibrinogen scaffolds are specifically immobilized on the APTES layer. Alternatively, immobilized nanofibrous fibrinogen scaffolds can be generated on a substrate that comprises at least one surface composed of a metal, such as of gold, or that comprises at least one surface composed of a polymer matrix, such as polylactide (PLA).

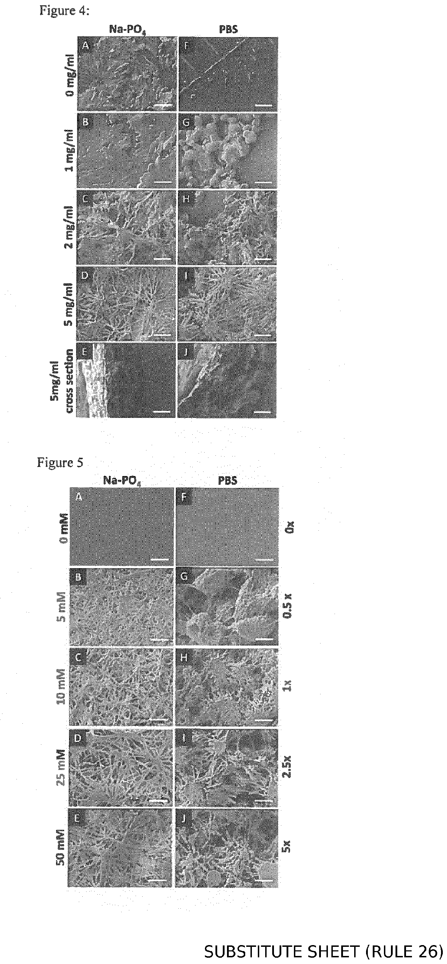

Figure 4. SEM images of nanofibrous fibrinogen scaffolds, which were prepared using varying protein concentrations and adding either 25 mM Na-PO₄ buffer (A to D) or 2.5x PBS (F to I) at pH 7.4. Below fibrinogen concentrations of 2 mg/ml nanofibers could not be assembled (A, B and F, G). With concentrations of 2 mg/ml fibrinogen in the sample volume or higher protein concentrations fiber formation was observed (C, D and H, I). With increasing protein concentration the fibrous scaffolds were found to be more dense. Cross-sections of scaffolds prepared with 5 mg/ml fibrinogen were additionally imaged for 25 mM Na-PO₄ buffer (E) and 2.5x PBS (J). Both cross-sections show a scaffold thickness of several micrometers. Scale bars represent 2 µm.
Figure 5. SEM images of self-assembled fibrinogen scaffolds prepared with varying salt concentrations. Fibrinogen nanofibers were assembled by drying a 5 mg/ml fibrinogen solution in the presence of either Na-PO₄ buffer (B to E) or PBS (G to J), which were both at pH 7.4. Without the presence of any salt buffer no fiber formation was observed (A and F). For all other salt concentrations fibrinogen reproducibly assembled into nanofibers. Scale bars represent 2 µm.

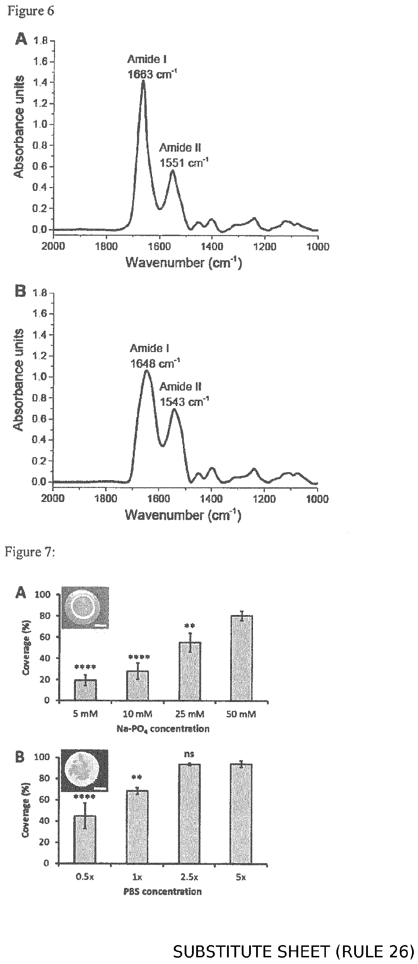

Figure 6. Representative FTIR spectra of different fibrinogen samples prepared on gold surfaces: (A) planar fibrinogen prepared with 5 mg/mL fibrinogen solution, (B) fibrinogen nanofibers prepared via self assembly of 5 mg/ml fibrinogen solution with 2.5× PBS (pH 7.4). Both spectra show the amide I and amide II regions, which are sensitive to protein superstructure. With nanofiber formation in the presence of PBS the peak positions were shifted towards lower wave numbers as compared to the spectra of planar fibrinogen.
Figure 7. Coverage of 15 mm glass slides with fibrinogen nanofibers increases with increasing salt concentration. Fibrinogen nanofibers were prepared by drying a 5 mg/ml fibrinogen solution in the presence of different concentrations of either (A) Na-PO₄ buffer or (B) PBS. The coverage of the 15 mm glass substrates was measured using a USB microscope. Data represent means and standard deviations of three independently prepared samples for each salt concentration. The average coverages of the samples with the highest concentration were compared to the other conditions by ANOVA and are indicated by asterisks (****p < 0.0001, **p < 0.001, ns: not significant). Insets show overview images of the 15 mm glass slides with 5 mg/ml fibrinogen dried in the presence of 25 mM Na-PO₄ buffer or 2.5x PBS, respectively. Scale bars in insets represent 5 mm.

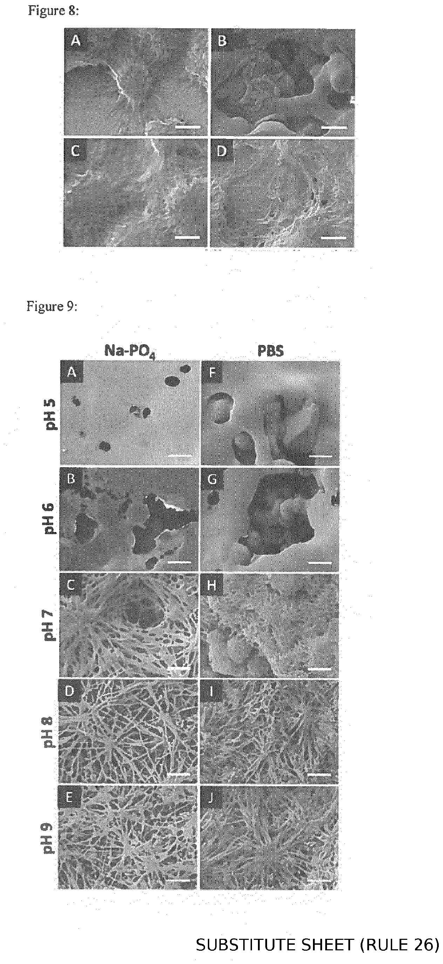

Figure 8. SEM images of fibrinogen nanofibers, which were assembled using different PBS components. 5 mg/ml fibrinogen were dried in the presence of (A) 375 mM NaCl, (B) 10 mM KCl, (C) 375 mM KCl or (D) 25 mM K-PO₄ buffer. Nanofibers could be assembled in all buffers, if the salt concentration was at least 25 mM. No fibrinogen fibers formed with 10 mM KCl. Fiber morphology was less defined when single PBS components were used. Scale bars represent 2 µm.
Figure 9. SEM images of fibrinogen nanofibers, which were assembled by drying a 5 mg/ml fibrinogen solution in either 25 mM Na-PO₄ buffer (A to E) or 2.5x PBS (F to J) under varying pH conditions. In both buffers the pH was varied from 5 to 9. Fiber formation was only induced for pH 7 to 9 whereas more acidic pH ranges did not yield any nanofibers. Scale bars represent 2 µm.

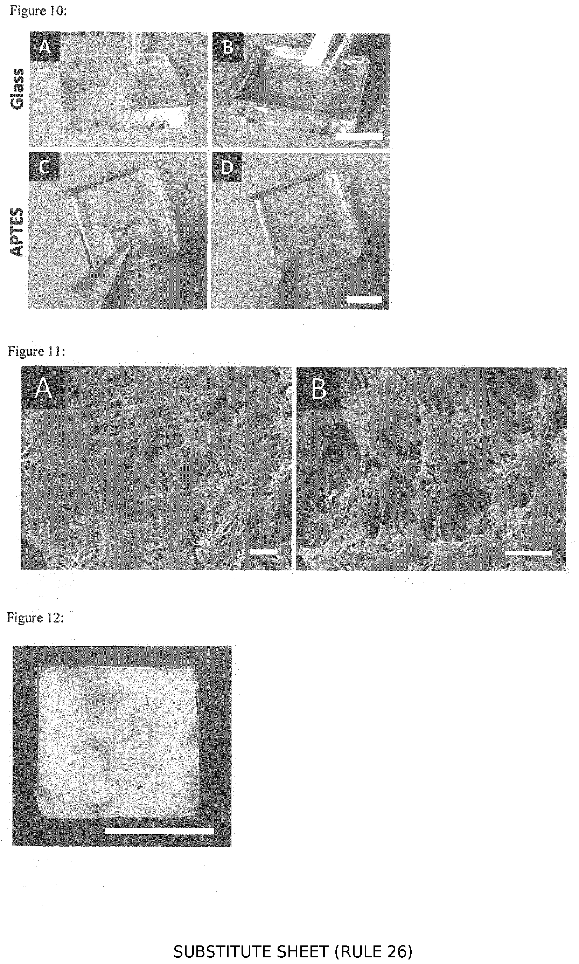

Figure 10. Controlled detachment or immobilization of fibrinogen nanofiber scaffolds depends on the underlying substrate material. Scaffolds were prepared using 5 mg/ml fibrinogen and 2.5× PBS. After fixation in formaldehyde vapor the samples were washed with water. On glass substrates the scaffolds detached immediately (A, B) whereas on APTES-modified substrates fibrinogen scaffolds stayed immobilized (C, D). Scale bars represent 1 cm.
Figure 11. SEM images of self-assembled fibrinogen scaffolds prepared in the presence of AEBSF. Fibrinogen nanofibers were assembled by drying a 5 mg/ml fibrinogen solution in the presence 2.5× PBS and 0.1 mM (A) or 1 mM (B) AEBSF. Even in the presence of the thrombin inhibitor AEBSF fiber formation was observed. Scale bars represent 2 µm.
Figure 12. Self-assembled fibrinogen scaffold with a total surface area of approximately 6 cm2. Fibrinogen nanofibers were assembled by drying a 5 mg/ml fibrinogen solution in the presence of 2.5× PBS on a glass slide. Scale bar represents 10 mm.

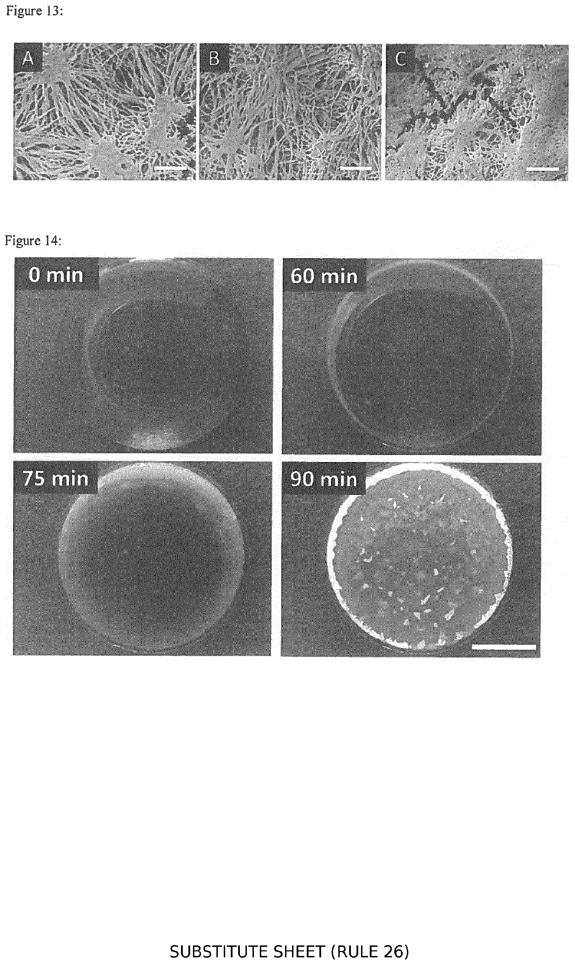

Figure 13. SEM images of self-assembled fibrinogen scaffolds prepared on different surfaces. Fibrinogen nanofibers were assembled by drying a 5 mg/ml fibrinogen solution in the presence of 2.5× PBS on (A) an APTES-modified surface, (B) a sputtered gold surface or (C) a polystyrene cell culture dish. Fibrinogen fibers formed on both substrates when PBS was added. Scale bars represent 2 µm.
Figure 14. Fibrinogen scaffold preparation at different time points. Fibrinogen nanofibers were assembled by drying a 5 mg/ml fibrinogen solution in the presence of 2.5× PBS. After 60 min a change in turbidity was observed during drying, after 90 min macroscopic salt crystals were visible. Scale bar represents 5 mm.

### Examples

### Preparation of substrates and protein solutions

Round glass coverslips with a diameter of 15 mm (VWR, Darmstadt, Germany) and square glass slides (Gerhard Menzel GmbH, Braunschweig, Germany) were cleaned by 5 min immersion into H2SO5 (piranha solution). Piranha solution was freshly prepared by mixing 95% sulfuric acid (VWR) with 30% hydrogen peroxide solution (VWR) in a 3:1 ratio. After washing with deionized water from a TKA water purification system (Thermo Fisher Scientific, Schwerte, Germany) the activated glass coverslips were stored in deionized water and dried under nitrogen flow directly before further use. Moreover, the inventors studied the self assembly of fibrinogen on polystyrene petri dishes (Sarstedt, Numbrecht, Germany) and on glass, which was sputter-coated with a 25 nm gold layer in a Bal-Tec SCD 005 system (Leica Microsystems, Wetzlar, Germany). The inventors also analyzed fibrillogenesis of fibrinogen on glass slides, which were modified with (3-Aminopropyl)triethoxysilane (APTES) by immersion in an ethanol solution (VWR) containing 5% APTES (Sigma) for 16 h at room temperature. Fibrinogen stock solutions were prepared by dissolving 10 mg/ml fibrinogen (100% clottable Merck, Darmstadt, Germany) in a 10 mM ammonium carbonate solution (Roth, Karlsruhe, Germany). The fibrinogen stock solution was dialyzed against 10 mM ammonium carbonate solution overnight using cellulose membrane dialysis tubing with 14 kDa cut-off (Sigma, Steinheim, Germany) to remove low molecular weight compounds. In control experiments varying concentrations of the thrombin inhibitor 4-(2-Aminoethyl)benzensulfonylfluorid (AEBSF, Sigma) were added to the buffer system to inactivate any potential thrombin residues in the fibrinogen stock.

### Self assembly of fibrinogen nanofibers

The standard procedure to prepare nanofibrous fibrinogen scaffolds was to deposit 100 µl of the 10 mg/ml fibrinogen stock solution on piranha-cleaned glass slides. Subsequently, fiber formation was induced by adding 100 µl of either 5x PBS solution (Thermo Fisher) or 50 mM sodium phosphate (Na-PO₄) buffer (Roth, Karlsruhe, Germany), which were both at pH 7.4. The final concentration in the samples was 5 mg/ml fibrinogen and 5 mM NH₄HCO₃ in either 2.5× PBS or 25 mM Na-PO₄ buffer. For planar reference samples 100 µl of deionized water was added to the fibrinogen instead of PBS or Na-PO₄ buffer. All samples were subsequently dried overnight in ambient conditions. To analyze the influence of varying buffer conditions on the fibrillogenesis process the inventors later used variations of this standard procedure by adjusting the fibrinogen or buffer concentration. The inventors also studied the dependence of fibrillogenesis on varying pH in PBS or Na-PO₄ buffers, which were prepared by adding different ratios of NaH₂PO₄ and Na2HPO₄ (Roth). NaCl (AppliChem, Darmstadt, Germany) was added to the Na-PO₄ buffers to obtain PBS with varying pH. To investigate the influence of different salt ions on the fiber formation the inventors also prepared samples with 375 mM NaCl (AppliChem), 10 and 375 mM KCl (Roth, Karlsruhe, Germany) or 25 mM K-PO₄ buffer (pH 7.4, AppliChem).

### Free-standing and immobilized fibrinogen scaffolds

For the preparation of free-standing scaffolds the inventors crosslinked self-assembled fibrinogen nanofibers on glass slides overnight using a sealed chamber, which contained 200 µl of 37% formaldehyde (AppliChem). Afterwards, fixated scaffolds were dried for 1 hour, followed by a washing step in deionized water. To fabricate immobilized fibrinogen scaffolds the inventors carried out the same procedure on APTES-coated glass, gold and polystyrene petri dishes.

### Microscopy analysis of nanofibrous fibrinogen scaffolds

The coverage of glass slides with dried fibrinogen nanofibers was analyzed using a USB universal microscope (Meade Instruments, Rhede, Germany) using an 20x magnification and brightfield imaging. For morphological analysis dried fibrinogen samples were sputter-coated with 7 nm of gold using a Bal-Tec SCD 005 sputter system (Leica Microsystems). Scanning electron microscopy (SEM) analysis was carried out with a Zeiss Auriga field emission device (Carl Zeiss, Oberkochen, Germany) at acceleration voltages of 3 kV. Surface coverage and fiber diameters were analysed using the open source software ImageJ. Differences in surface coverage were analyzed by ANOVA followed by the Tukey post hoc-test.

### Fourier-transform infrared reflective absorption spectroscopy

Fourier-transform infrared reflective absorption spectroscopy (FTIR) analysis of fibrinogen scaffolds on AU-coated glasses was conducted using a Bruker Vertex 70 with IR Scope II (Broker, Ettlingen, Germany). First, round glass coverslips with a diameter of 15 mm (VWR, Darmstadt, Germany) were sputter-coated with an adhesion layer of 5 nm of chrome, followed by 50 nm of gold using an EM ACE600 high vacuum sputter coater (Leica Microsystems, Wetzlar, Germany). Subsequently, fibrinogen scaffolds were prepared on the Au layers using a final protein concentration of 5 mg/ml and either 2.5× PBS with 5 mM NH₄HCO₃ for fibrous scaffolds or 5 mM NH₄HCO₃ for planar fibrinogen samples. Each sample was dried under ambient conditions and measured in 15 different points in a zig-zag pattern. Spectra were recorded at 4 cm-1 resolution and 60 scans were averaged per measurement. The reference spectrum was measured against air and subtracted from the obtained spectra. The spectra were processed and evaluated by the Bruker software package OPUS. After subtraction of water vapour absorbencies (atmosphere compensation) the resulting spectra were smoothed by a 7-17 point Savitsky-Golay function, depending on the quality of the data and baseline corrected by rubber band baseline correction. Amide I and amide II peak positions were determined by peak integration using the Origin 9.0 software (OriginLab Northampton, USA).

### Effect of fibrinogen concentration on nanofiber assembly

When 25 mM Na-PO₄ buffer or 2.5× PBS were added to fibrinogen dissolved in NH₄HCO₃ and samples were dried under ambient conditions for SEM analysis the inventors observed the formation of nanofibers in dependence of the fibrinogen concentration. Only salt crystals were found when no protein was present in the respective buffer. With fibrinogen concentrations of 1 mg/ml and lower only globular aggregates were found in both buffer systems. For both buffers the inventors observed that a minimum concentration of 2 mg/ml fibrinogen in solution was necessary to induce nanofiber assembly upon drying. The nanofibrous scaffolds became more dense when the fibrinogen concentration was increased further up to 5 mg/ml. The density of the self-assembled fibrous fibrinogen networks was comparable to previous electrospun fibrinogen mats.

To exclude the possible involvement of residual thrombin in the fibrinogen stock solution, the inventors added 0.1 and 1 mM of the thrombin inhibitor AEBSF to the fibrinogen solution and dried it in 2.5× PBS. It can be seen clearly that fibrinogen fibers also formed in 2.5× PBS when AEBSF was present. Cross sections of dense nanofiber scaffolds, which were prepared with the highest concentration of 5 mg/ml fibrinogen, revealed that the nanofibrous scaffolds had a thickness of 3 to 5 µm.

### Influence of varying buffer concentration on fibrinogen nanofiber assembly

To study the mechanism of salt-induced fiber formation further the inventors varied the concentration of Na-PO₄ buffer and PBS, in which the protein was dried. In a control experiment without the presence of any salts no fibers formed and only planar films of fibrinogen were obtained. With low salt concentrations of only 5 mM Na-PO₄ buffer or 0.5x PBS fibrinogen already assembled into nanofibers upon drying. When salt concentrations were increased even further from 10 mM to 50 mM Na-PO₄ buffer and from 1x to 5x PBS fibrinogen also assembled into nanofibers. In all fibrous assemblies the diameter of single fibers varied between 100 and 300 nm and was independent of the respective buffer or salt concentration.

### Structural analysis of fibrinogen fibrillogenesis

To study the observed morphological changes upon salt-induced fiber assembly in more detail the inventors carried out FTIR analysis of planar and fibrous fibrinogen scaffolds on gold. The FTIR spectra were characterized within a range of vibration frequencies from 2000 to 1000 cm-1 as proteins exhibit characteristic bands in this spectral range. Figure 6 shows representatives FTIR spectra of planar fibrinogen samples and of fibrinogen nanofibers. Two well defined and typical bands between 1600 - 1700 cm-1 (amide I) and between 1500 -1600 cm-1 (amide II) were observed in both samples. For the planar samples the amide I band was centered at 1663 cm-1 while for fibers the peak position shifted to 1648 cm-1. The amide II band also shifted to lower wave number values from 1551 cm-1 for planar fibrinogen to 1543 cm-1 for fibrinogen nanofibers. Furthermore, it can be seen in Fig. 6 that the peak shape of planar fibrinogen differed from the peaks obtained for fibrinogen nanofibers. The peaks of planar fibrinogen exhibited a more narrow shape and an amide I/amide II ratio of 2.45. In contrast, fibrinogen nanofibers led to broader peaks and a lower amide I/amide II ratio of 1.50.

When comparing planar fibrinogen samples to nanofibrous fibrinogen biomaterial, it is apparent that planar fibrinogen samples appear to be white, while nanofibrous fibrinogen biomaterial as generated by salt-induced assembly (such as by addition of PBS) appears to have a whitish color. When analyzing a cross-section of a planar fibrinogen sample prepared according to this invention, the planar fibrinogen layers are very thin and smooth, even at a thickness of the entire scaffold of about 1,3 µm. Accordingly, the transparency of the planar fibrinogen samples can be explained by their smooth structure. This smoothness of the planar fibrinogen samples prepared according to this invention can be observed for scales of up to multiple centimeters.

Interestingly, when the inventors increased the salt concentration in both buffer systems the inventors observed an increase in the overall coverage of fibrinogen nanofibers on the glass substrates as tested. ANOVA analysis confirmed that the lower buffer concentrations, i.e. 5 mM to 25 mM Na-PO₄ and 0.5 to 1x PBS, resulted in a significantly lower coverage with fibrinogen nanofibers (Fig. 7). For Na-POq. concentrations up to 50 mM a maximum coverage of 80% was achieved on 15 mm large coverslips (Fig 7A). With PBS the inventors even obtained a maximum coverage of 95% with concentrations of 2.5x and 5x PBS, respectively (Fig 7B). Hence, PBS-induced fibrillogenesis on glass slides resulted in fibrinogen scaffolds with a size of approximately 1.75 cm2. On larger glass substrates the inventors could also fabricate nanofibrous scaffolds with a surface area of approximately 6 cm2 (Fig. 12). Moreover, using the inventors' self assembly procedure the inventors could prepare nanofibrous fibrinogen scaffolds on other biocompatible surfaces like APTES, gold and polystyrene (Fig. 13). The fiber morphology on these substrates was comparable to fibers assembled on glass.

### Effect of individual PBS components on fibrinogen nanofiber assembly

So far, fiber formation was only observed when fibrinogen was dried in the presence of Na-PO₄ buffer or PBS. The 2.5x PBS used in the inventors' initial experiments contained 25 mM Na-PO₄ buffer, 350 mM NaCl and 6.7 mM KCl. To elucidate whether any of these PBS components induce fiber formation on their own the inventors analyzed fiber assembly using 375 mM NaCl and 10 mM KCl, respectively (Fig. 8). Fiber formation was observed with 375 mM NaCl while the presence of 10 mM KCl did not yield any nanofibers. However, when the KCl concentration was increased to 375 mM, fiber formation could also be induced. Overall, in these buffer systems the fiber morphology was less defined and fibers were unevenly distributed as compared to fibers assembled in Na-PO₄ buffer or PBS. In comparison to the inventors' standard routine with 25 mM Na-PO₄-buffer the inventors carried out an additional experiment in the presence of 25 mM K-PO₄ buffer. Fig. 8D clearly shows that this buffer also induced self-assembly of fibrinogen into nanofibers.

### Nanofiber assembly under varying pH conditions

Since the addition of Na-PO₄ buffer or PBS (both at pH 7.4) to the inventors' fibrinogen stock in ammonium carbonate solution (pH 8.6) resulted in a pH shift from the slightly alkaline to physiological pH the inventors were interested in the influence of different pH values on the self assembly process. When 5 mg/ml fibrinogen were used and the pH of 25 mM Na-PO₄ buffer and 2.5× PBS was adjusted to 5 or 6, respectively, no fiber formation could be induced (Fig. 9). Nevertheless, when Na-PO₄ buffer and PBS with a pH between 7 and 9 were added to the fibrinogen solution the inventors observed fiber assembly in all samples.

### Free-standing and immobilized fibrinogen scaffolds

When fibrinogen nanofibers were assembled on glass slides an additional fixation step in formaldehyde vapor was introduced followed by washing in water. After this treatment the inventors observed immediate scaffold detachment from the underlying glass surface. When APTES was used as substrate for self-assembled fibrinogen nanofibers the scaffolds stayed immobilized on the silane surface upon cross-linking and washing. Immobilized fibrinogen scaffolds were also obtained when fibrinogen fibers were assembled on gold and polystyrene surfaces prior to fixation and washing. SEM analysis of fixated fibrinogen scaffolds after the final washing step revealed that the nanofiber morphology was preserved during this procedure for both - immobilized and free-floating - fibrinogen assemblies. Interestingly, without a fixation in formaldehyde vapor the inventors observed that the inventors' self-assembled fibrinogen fibers dissolved again upon washing.

**Table 1:**

| | Ionic strength (mM) | |
|---|---|---|
| pH | 25 mM Na-PO₄ | 2.5x PBS |
| 5 | 26 | 376 |
| 6 | 32 | 382 |
| 7 | 62 | 412 |
| 8 | 72 | 422 |
| 9 | 74 | 424 |

The novel self assembly process of this invention to prepare nanofibrous fibrinogen scaffolds only requires the addition of salt solutions like Na-PO₄ buffer or PBS to induce fibrillogenesis in vitro. Interestingly, when the protease inhibitor AEBSF was added to the self assembly system, the inventors could also observe nanofiber formation. This result clearly indicates that nanofibrous fibrinogen scaffolds did not assemble due to possible thrombin residues in the purchased fibrinogen. Hence, the inventors assume that the formation of self assembled fibrinogen fibers was solely induced by the presence of salt buffer in combination with a drying step. Various enzyme-independent methods have previously been used to induce fibrillogenesis of fibrinogen in *vitro* - also in the presence of Na-PO₄ buffer or PBS at pH 7.4.

The inventors observed that even salt concentrations as low as 5 mM Na-PO₄ were sufficient to induce self assembly of fibrinogen into nanofibers without any nanostructured substrates or acidic buffer conditions. The presence of single PBS components also induced successful fiber formation. Therefore, the inventors' results strengthen the hypothesis that an increase in salt ion concentration and particularly the presence of Na+ or K+ ions are responsible for in *vitro* fibrillogenesis of fibrinogen. Interestingly, the inventors could achieve salt-induced assembly of fibrinogen fibers over a broad pH range even in alkaline pH. Previously, an acidic pH of 2 was used to induce fibrillogenesis. It was suggested that this pH change induced a conformational change, which resulted in aggregation of fibrinogen molecules and in fiber formation. Furthermore, pH changes are known as the major driving force behind the well-understood self assembly mechanism of the ECM protein collagen into nanofibers. However, the pH change in the experimental system was comparably small or not present at all. The inventors therefore conclude that the small pH shift from 8.6 to 7.4, which was used in most of the inventors' experiments, was not the major driving force of fibrinogen fiber formation since fibers were also formed when both buffer systems were at pH 7.4. It had previously been reported that fibrinogen at pH 7.4 has a negative net charge. This finding supports the hypothesis of positively charged Na+ or K+ ions interacting with negatively charged fibrinogen molecules. Interestingly, no fiber formation was observed at pH lower than 7 for both buffer systems, which could be a result of a decreased net charge of the fibrinogen. Na-PO₄ buffers with low pH have a lower ionic strength compared to Na-PO₄ buffers with the same concentration at a higher pH (Table 1). However, the inventors did not observe any fiber formation when PBS with pH 5 or 6 was used, which had a much higher ionic strength than Na-PO₄ buffer at the same pH. Hence, the inventors conclude that lack of fiber formation is caused by the pH itself and not due to a difference in the ionic strength. When the inventors characterized planar and fibrous fibrinogen scaffolds with FTIR spectroscopy the inventors observed differences in the amide I and II bands. Both amide peaks of fibrous fibrinogen were shifted towards lower wave numbers as compared to the spectra of planar fibrinogen. Since the amide I vibration arises mainly from the C=O stretching vibration it is very sensitive to changes in the secondary structure. Hence, the pronounced amide I band shift of 15 cm-1 indicated that fibrinogen might be arranged in two different conformations as previously described for adsorbed fibrinogen films by Liedberg et *al.* Moreover, the overall peak shape, intensity and the ratio between amide I and amide II bands indicated that changes in the protein conformation occurred between planar and nanofibrous fibrinogen scaffolds, i.e. without and with the presence of salt in the fibrinogen solution. The peaks of planar fibrinogen were very narrow with an amide I/amide II ratio of 2.45. In contrast, fibrinogen fibers exhibited a broader peak shape with a lower amide I/amide II ratio of 1.50. These clear differences between the spectra suggest that planar and nanofibrous fibrinogen scaffolds possess different protein conformations as it was previously shown for fibrinogen adsorption on self assembled monolayers.

The inventors also observed fiber assembly of fibrinogen on hydrophilic surfaces like piranhacleaned glass, APTES or gold. Hence, the surface energy of the underlying substrate did not pose any limitation for the successful fiber formation in the salt-induced self assembly process of this invention as it had been previously reported for other methods. When fibrinogen solutions were monitored while drying in 2.5× PBS the inventors noticed a change from initially transparent to more turbid solutions towards the later stage of the drying process (Fig. 14). No change in turbidity was observed when planar fibrinogen samples were prepared by adding deionized water to fibrinogen dissolved in NH4HCO3.

Interestingly, the drying step influences the fibrinogen fibrillogenesis. When drying becomes the driving factor in fiber assembly one also has to consider the local increase in protein concentration and ionic strength in the solution during fibrillogenesis. Since the inventors observed that fibrinogen only assembled into nanofibers with protein concentrations of 2 mg/ml or higher, this finding strengthens the hypothesis that drying is crucial in salt-induced fibrillogenesis of fibrinogen. Based on observations the inventors assume that the detected change in turbidity occurred due to the lower solubility threshold of fibrinogen in comparison to the surrounding salt buffer. Hence, the inventors now suggest the following mechanism to be responsible for fiber assembly in salt buffers: during drying in the presence of salt ions fibrinogen molecules change their conformation and are oriented. Due to the gradual increase in protein concentration upon drying oriented molecules assemble into fibrils, and fibrillogenesis is induced. Interestingly, when dense nanofibrous assemblies were formed by salt-induced fibrillogenesis with 5 mg/ml fibrinogen the inventors mostly observed a characteristic "star shape morphology" where the fibers reticulated from individual center points. Therefore, the inventors assume that short fibrils function as individual nucleation sites, which subsequently trigger the assembly of further oriented fibrinogen molecules into nanometer-sized fibers upon drying.

The novel biofabrication process of this invention of salt-induced self assembly reproducibly yielded dense fibrinogen scaffolds with a very high nanofiber yield. Fiber diameters ranged from 100 to 300 nm, which is much larger in comparison to fibers, which were previously formed on hydrophobic surfaces with diameters between 2 and 20 nm. The dimensions of the self-assembled fibrinogen fibers were rather in the range of electrospun fibrinogen fibers, which exhibited diameters between 80 and 700 nm. With regard to future tissue engineering applications it will be advantageous that the dimensions of the self-assembled fibrinogen fibers are well comparable to nanofibers in the native ECM, which vary from 50 to 500 nm, and with the dimensions of natural fibrin fibers, which range from 20 to 200 nm.

With overall diameters of several centimeters the scaffold size of the self-assembled fibrinogen networks is also comparable with the dimensions of electrospun fibrinogen mats. Nevertheless, electrospinning required fibrinogen concentrations of 100 to 200 mg/ml to prepare nanofibrous scaffolds with overall dimensions in the centimeter range. On the contrary, the self assembly approach of this invention only required extremely low fibrinogen concentrations starting from 2 mg/ml. With such low protein concentrations the new self assembly routine of this invention will be a very cost-efficient procedure to prepare nanofibrous fibrinogen scaffolds on a large scale. Moreover, self-assembled scaffolds prepared from only 5 mg/ml fibrinogen were 3 to 5 µm thick whereas electrospun fibrinogen scaffolds prepared with much higher protein concentrations were approximately 0.7 mm thick. With regard to future scaffold applications in regenerative medicine it will be very interesting to study how the thickness of self-assembled fibrinogen scaffolds can be increased even further by adjusting the protein concentration and other parameters in the self assembly process. In contrast to previous approaches the new self assembly routine of this invention facilitates fibrillogenesis of fibrinogen in an enzyme-free environment. Another advantage of salt-induced self assembly is the fact that it does not require any high electric fields or denaturing organic solvents like 1,1,1,3,3,3- hexafluoro-2-propanol, which might impede the biological functionality of the resulting fibrinogen fiber scaffolds. The versatile choice of substrate materials to prepare fibrinogen nanofibers by salt-induced self assembly is another important advantage, which will make the new biofabrication strategy highly attractive for the modification of implant coatings or novel wound dressings.

Importantly, the use of different substrate materials also introduced the unique possibility to prepare either free-standing or immobilized fibrinogen scaffolds on demand. By adding a fixation in formaldehyde vapor to preserve the nanofiber morphology upon washing the inventors were able to induce controlled detachment of the 3D-scaffolds from glass slides. On the other hand, using silanized glass, polystyrene or gold surfaces as underlying substrate enabled the inventors to specifically immobilize self-assembled fibrinogen scaffolds on these surfaces upon fixation. This versatile and well-controllable routine is easy to use and can be implemented with standard lab equipment and low-cost consumables. Using only physiological buffer systems the novel self assembly method offers a powerful biofabrication platform to prepare immobilized or free-standing fibrinogen scaffolds for various biomedical applications.

Nano- or microfibrous scaffolds are prepared from aqueous fibrinogen solutions (i.e, at physiological pH) on glass substrates using different preparation methods (for instance salt-induced self-assembly for nanofibers or re-hydration of adhered fibrinogen films for microfibrous assemblies). If the fibrous scaffolds are prepared on piranha-cleaned glass, a subsequent fixation step in paraformaldehyde vapor followed by washing in aqueous solution facilitates controlled detachment of the fibrous scaffolds. If a silanization of the glass surface with (3-Aminopropyl)triethoxysilane (APTES) is introduced into the procedure prior to the respective fiber preparation method, the final fixation and subsequent washing will result in fibrous fibrinogen scaffolds, which are immobilized on the substrate. Likewise, the APTES modification can be used to immobilize planar fibrinogen on the underlying substrate.

Importantly, nano- or microfibrous fibrinogen scaffolds are prepared from fibrinogen solutions using different methods. Salt-induced self-assembly is, for instance, used to prepare fibrinogen nanofibers, or re-hydration of adsorbed fibrinogen films in aqueous buffer is used to produce microfibrous fibrinogen scaffolds. After a drying step the fibrous scaffolds are fixated in a chamber with formaldehyde vapour, followed by repeated washing steps in aqueous buffer. When fibrous scaffolds are prepared on bare glass the fixation and repeated washing steps induce controlled detachment of the fibrous scaffolds from the underlying glass. This procedure yields three-dimensional, free-floating fiber scaffolds in solution, which
can be transferred, for instance, to cell cultures or injured tissue to facilitate wound healing. On the APTES-modified glasses the fibrous scaffolds stay immobilized upon washing and can be used as cell or tissue scaffold.

Accordingly, the inventors succeeded in the first-time preparation of either free-standing or immobilized fibrinogen scaffolds. This unique feature could be realized by tailoring the underlying substrate material and by introducing a customized fixation and washing procedure after the fiber assembly. Fibrillogenesis could be induced with a fibrinogen concentration of at least 2 mg/ml in a pH regime of 7 to 9. Fiber diameters ranged from 100 to 300 nm, thus resembling native fibrin and ECM protein fibers. By adjusting the salt concentration the inventors could prepare fibrinogen scaffolds with overall dimensions in the centimeter range and a thickness of 3 to 5 µm. Using FTIR analysis the inventors observed peak shifts of the amide bands for fibrinogen nanofibers in comparison to planar fibrinogen, which indicates changes in the secondary structure. Since fibrillogenesis was only induced upon drying when salt ions were present the inventors assume that protein molecules were locally oriented in the respective buffers, which - in combination with the observed conformational changes - led to the assembly of individual molecules into fibers. In summary, the inventors' novel self assembly process offers a simple and well controllable method to prepare large-scale 3D-scaffolds of fibrinogen nanofibers under physiological conditions. The unique possibility to chose between free-standing and immobilized scaffolds makes the inventors' novel biofabrication process highly attractive for the preparation of versatile tissue engineering scaffolds.

Using scanning electron microscopy the inventors observed that different buffers including phosphate buffered saline and sodium phosphate reproducibly yielded dense fiber networks on bare and silanized glass surfaces, gold as well as polystyrene upon drying. Finally, the three-dimensional, free-floating fiber scaffolds can be tested in vivo and in vitro, such as in cell culture experiments using fibroblasts or endothelial cells.

## Claims

1. A method of generating a fibrous fibrinogen biomaterial, comprising
a) Providing a substrate,
b) Optionally, cleaning said substrate,
c) Optionally, modifying of at least one surface of said substrate,
d) Adding a fibrinogen solution to said substrate, or submerging said substrate in said fibrinogen solution,
e) Adding a salt buffer and/or an aqueous buffer and/or water to said substrate, or submerging said substrate in said salt buffer and/or said aqueous buffer and/or said water,
f) Optionally, drying said substrate,
g) Optionally, adding a salt buffer and/or an aqueous buffer and/or water to said substrate, or submerging said substrate in said salt buffer and/or said aqueous buffer and/or said water,
h) Optionally, drying said substrate,
i) Optionally, performing one or more repetitions of steps g) to h),
j) Fixating said substrate, and
k) Washing said substrate,
thereby generating said fibrous fibrinogen biomaterial.

2. The method according to claim 1, wherein said generated fibrous fibrinogen biomaterial is composed of three-dimensional fibrinogen fibers, optionally wherein said fibrous fibrinogen biomaterial can be used as a scaffold.

3. The method according to claim 2, wherein said fibrinogen fibers have a diameter of between 0.1 nm and 10000 nm, preferably between 1 nm and 1000 nm, more preferably between 10 nm and 500 nm, and most preferably between 100 nm and 200 nm.

4. The method according to any one of claims 1 to 3, wherein said fibrous fibrinogen biomaterial to be generated has a hydrodynamic radius of at least 0.00001 mm, preferably of at least 0.001 mm, more preferably of at least 0.01 mm, further preferably of at least 0.1 mm, even more preferably of at least 1 mm, and most preferably of at least 10 mm.

5. The method according to any one of claims 1 to 4, wherein said substrate as provided in a) is composed of a solid material, such as a solid glass material, optionally wherein said substrate comprises at least one surface composed of a metal or a polymer, preferably wherein said metal is selected from gold, silver, copper, gold-platted copper, tinned copper, aluminum, platinum, indium, tungsten, beryllium, gallium, lithium, calcium, magnesium, zinc, titanium, zirconium, hafnium, and mixtures thereof, more preferably wherein said metal is gold, or wherein said polymer is preferably selected from polylactide (PLA), polystyrol, polybutyrate adipate terephthalate (PBAT), and mixtures thereof, more preferably wherein said polymer is polylactide (PLA).

6. The method according to any one of claims 1 to 5, wherein said fibrinogen solution as added in step d) has a fibrinogen concentration of between 0.01 and 1000 mg/ml, preferably between 0.1 and 100 mg/ml, more preferably between 0.5 and 50 mg/ml, and most preferably between 1 and 5 mg/ml.

7. The method according to any one of claims 1 to 6, wherein said fibrinogen solution as added in step d) is an aqueous fibrinogen solution.

8. The method according to any one of claims 1 to 7, wherein said salt buffer comprises at least one component selected from sodium phosphate, sodium chloride, acetic acid, ammonium carbonate, ammonium phosphate, boric acid, citric acid, lactic acid, phosphoric acid, potassium chloride, potassium citrate, potassium metaphosphate, potassium phosphate (monobasic), sodium acetate, sodium citrate, sodium lactate, sodium phosphate (dibasic), sodium phosphate (monobasic), and mixtures thereof.

9. The method according to any one of claims 1 to 8, wherein said salt buffer is phosphate-buffered-saline (PBS), optionally wherein said PBS comprises sodium chloride, potassium chloride, sodium phosphate, and potassium phosphate.

10. The method according to any one of claims 1 to 9, wherein said fixating step j) is performed by immersing said substrate in a fixation agent selected from paraformaldehyde, formaldehyde, glutaraldehyde, mercury oxide, lead oxide, osmium oxide, trichloroacetic acid, acetic acid, and mixtures thereof, preferably wherein said fixation agent is paraformaldehyde.

11. The method according to any one of claims 1 to 10, wherein said washing step k) is performed in an aqueous solution.

12. A fibrous fibrinogen biomaterial, produced by a method according to any of claims 1 to 11.

13. A fibrous fibrinogen biomaterial according to claim 12 or a composition comprising said fibrous fibrinogen biomaterial for use as a medicament.

14. A fibrous fibrinogen biomaterial according to claim 12 or a composition comprising said fibrous fibrinogen biomaterial for use in the treatment of a degenerative disease, a wound, a dermal disease, a vessel damage, and/or a skin damage.

## Patentansprüche

1. Verfahren zum Erzeugen eines faserförmigen Fibrinogen-Biomaterials, umfassend
a) Bereitstellen eines Substrats,
b) gegebenenfalls Reinigen des Substrats,
c) gegebenenfalls Modifizieren mindestens einer Oberfläche des Substrats,
d) Zugeben einer Fibrinogenlösung zu dem Substrat oder Eintauchen des Substrats in die Fibrinogenlösung,
e) Zugeben eines Salzpuffers und/oder eines wässrigen Puffers und/oder von Wasser zu dem Substrat oder Eintauchen des Substrats in den Salzpuffer und/oder den wässrigen Puffer und/oder das Wasser,
f) gegebenenfalls Trocknen des Substrats,
g) gegebenenfalls Zugeben eines Salzpuffers und/oder eines wässrigen Puffers und/oder von Wasser zu dem Substrat oder Eintauchen des Substrats in den Salzpuffer und/oder den wässrigen Puffer und/oder das Wasser,
h) gegebenenfalls Trocknen des Substrats,
i) gegebenenfalls Durchführen einer oder mehrerer Wiederholungen der Schritte g) bis h),
j) Fixieren des Substrats und
k) Waschen des Substrats,
wodurch das faserförmige Fibrinogen-Biomaterial erzeugt wird.

2. Verfahren nach Anspruch 1, wobei das erzeugte faserförmige Fibrinogen-Biomaterial aus dreidimensionalen Fibrinogenfasern besteht, wobei das faserförmige Fibrinogen-Biomaterial gegebenenfalls als Gerüst verwendet werden kann.

3. Verfahren nach Anspruch 2, wobei die Fibrinogenfasern einen Durchmesser zwischen 0,1 nm und 10000 nm, vorzugsweise zwischen 1 nm und 1000 nm, mehr bevorzugt zwischen 10 nm und 500 nm und am meisten bevorzugt zwischen 100 nm und 200 nm aufweisen.

4. Verfahren nach einem der Ansprüche 1 bis 3, wobei das zu erzeugende faserförmige Fibrinogen-Biomaterial einen hydrodynamischen Radius von mindestens 0,00001 mm, vorzugsweise von mindestens 0,001 mm, mehr bevorzugt von mindestens 0,01 mm, weiter bevorzugt von mindestens 0,1 mm, noch mehr bevorzugt von mindestens 1 mm und am meisten bevorzugt von mindestens 10 mm aufweist.

5. Verfahren nach einem der Ansprüche 1 bis 4, wobei das in a) bereitgestellte Substrat aus einem festen Material, wie einem festen Glasmaterial, besteht, wobei das Substrat gegebenenfalls mindestens eine Oberfläche umfasst, die aus einem Metall oder einem Polymer besteht, wobei das Metall vorzugsweise aus Gold, Silber, Kupfer, vergoldetem Kupfer, verzinntem Kupfer, Aluminium, Platin, Indium, Wolfram, Beryllium, Gallium, Lithium, Calcium, Magnesium, Zink, Titan, Zirconium, Hafnium und Mischungen davon ausgewählt ist, wobei das Metall mehr bevorzugt Gold ist, oder wobei das Polymer vorzugsweise aus Polylactid (PLA), Polystyrol, Polybutyratadipatterephthalat (PBAT) und Mischungen davon ausgewählt ist, wobei das Polymer mehr bevorzugt Polylactid (PLA) ist.

6. Verfahren nach einem der Ansprüche 1 bis 5, wobei die Fibrinogenlösung, wie in Schritt d) zugegeben, eine Fibrinogenkonzentration zwischen 0,01 und 1000 mg/ml, vorzugsweise zwischen 0,1 und 100 mg/ml, mehr bevorzugt zwischen 0,5 und 50 mg/ml und am meisten bevorzugt zwischen 1 und 5 mg/ml aufweist.

7. Verfahren nach einem der Ansprüche 1 bis 6, wobei die Fibrinogenlösung, wie in Schritt d) zugegeben, eine wässrige Fibrinogenlösung ist.

8. Verfahren nach einem der Ansprüche 1 bis 7, wobei der Salzpuffer mindestens eine Komponente umfasst, die aus Natriumphosphat, Natriumchlorid, Essigsäure, Ammoniumcarbonat, Ammoniumphosphat, Borsäure, Zitronensäure, Milchsäure, Phosphorsäure, Kaliumchlorid, Kaliumcitrat, Kaliummetaphosphat, Kaliumphosphat (einbasisch), Natriumacetat, Natriumcitrat, Natriumlactat, Natriumphosphat (zweibasisch), Natriumphosphat (einbasisch) und Mischungen davon ausgewählt ist.

9. Verfahren nach einem der Ansprüche 1 bis 8, wobei der Salzpuffer phosphatgepufferte Kochsalzlösung (PBS) ist, wobei das PBS gegebenenfalls Natriumchlorid, Kaliumchlorid, Natriumphosphat und Kaliumphosphat umfasst.

10. Verfahren nach einem der Ansprüche 1 bis 9, wobei der Fixierungsschritt j) durch Eintauchen des Substrats in ein Fixierungsmittel durchgeführt wird, das aus Paraformaldehyd, Formaldehyd, Glutaraldehyd, Quecksilberoxid, Bleioxid, Osmiumoxid, Trichloressigsäure, Essigsäure und Mischungen davon ausgewählt ist, wobei das Fixierungsmittel vorzugsweise Paraformaldehyd ist.

11. Verfahren nach einem der Ansprüche 1 bis 10, wobei der Waschschritt k) in einer wässrigen Lösung durchgeführt wird.

12. Faserförmiges Fibrinogen-Biomaterial, hergestellt durch ein Verfahren nach einem der Ansprüche 1 bis 11.

13. Faserförmiges Fibrinogen-Biomaterial nach Anspruch 12 oder Zusammensetzung, die das faserförmige Fibrinogen-Biomaterial umfasst, zur Verwendung als Medikament.

14. Faserförmiges Fibrinogen-Biomaterial nach Anspruch 12 oder Zusammensetzung, die das faserförmige Fibrinogen-Biomaterial umfasst, zur Verwendung bei der Behandlung einer degenerativen Erkrankung, einer Wunde, einer Hauterkrankung, eines Gefäßschadens und/oder eines Hautschadens.

## Revendications

1. Méthode de production d'un biomatériau fibreux à base de fibrogène, comprenant
a) la fourniture d'un substrat,
b) éventuellement un nettoyage dudit substrat,
c) éventuellement une modification d'au moins une surface dudit substrat,
d) l'addition d'une solution de fibrinogène au dit substrat ou la submersion dudit substrat dans ladite solution de fibrinogène,
e) l'addition d'un tampon salin et/ou d'un tampon aqueux et/ou d'eau au dit substrat, ou la submersion dudit substrat dans ledit tampon salin et/ou ledit tampon aqueux et/ou ladite eau,
f) éventuellement un séchage dudit substrat,
g) éventuellement l'addition d'un tampon salin et/ou d'un tampon aqueux et/ou d'eau au dit substrat, ou la submersion dudit substrat dans ledit tampon salin et/ou ledit tampon aqueux et/ou ladite eau,
h) éventuellement un séchage dudit substrat,
i) éventuellement la réalisation de une ou plusieurs répétitions des étapes g) à h),
j) une fixation dudit substrat et
k) un lavage dudit substrat,
produisant ainsi ledit biomatériau fibreux à base de fibrogène.

2. Méthode selon la revendication 1, où ledit biomatériau fibreux à base de fibrogène produit est composé de fibres de fibrinogène tridimensionnelles, éventuellement où ledit biomatériau fibreux à base de fibrogène peut être utilisé en tant qu'échafaudage.

3. Méthode selon la revendication 2, où lesdites fibres de fibrinogène ont un diamètre entre 0,1 nm et 10 000 nm, préférablement entre 1 nm et 1 000 nm, plus préférablement entre 10 nm et 500 nm, et le plus préférablement entre 100 nm et 200 nm.

4. Méthode selon l'une quelconque des revendications 1 à 3, où ledit biomatériau fibreux à base de fibrogène à produire a un rayon hydrodynamique d'au moins 0,00001 mm, préférablement d'au moins 0,001 mm, plus préférablement d'au moins 0,01 mm, davantage préférablement d'au moins 0,1 mm, encore plus préférablement d'au moins 1 mm et le plus préférablement d'au moins 10 mm.

5. Méthode selon l'une quelconque des revendications 1 à 4, où ledit substrat tel que fourni en a) est composé d'un matériau solide, tel qu'un matériau solide vitreux, éventuellement où ledit substrat comprend au moins une surface composée d'un métal ou d'un polymère, préférablement où ledit métal est sélectionné parmi l'or, l'argent, le cuivre, le cuivre plaqué d'or, le cuivre étamé, l'aluminium, le platine, l'indium, le tungstène, le béryllium, le gallium, le lithium, le calcium, le magnésium, le zinc, le titane, le zirconium, le hafnium et des mélanges de ceux-ci, plus préférablement où ledit métal est l'or, ou où ledit polymère est préférablement sélectionné parmi le polylactide (PLA), le polystyrol, le poly(adipate-co-téréphtalate de butylène) (PBAT) et des mélanges de ceux-ci, plus préférablement où ledit polymère est le polylactide (PLA).

6. Méthode selon l'une quelconque des revendications 1 à 5, où ladite solution de fibrinogène telle qu'ajoutée à l'étape d) a une concentration en fibrinogène entre 0,01 et 1 000 mg/ml, préférablement entre 0,1 et 100 mg/ml, plus préférablement entre 0,5 et 50 mg/ml et le plus préférablement entre 1 et 5 mg/ml.

7. Méthode selon l'une quelconque des revendications 1 à 6, où ladite solution de fibrinogène telle qu'ajoutée à l'étape d) est une solution aqueuse de fibrinogène.

8. Méthode selon l'une quelconque des revendications 1 à 7, où ledit tampon salin comprend au moins un composant sélectionné parmi les suivants : phosphate de sodium, chlorure de sodium, acide acétique, carbonate d'ammonium, phosphate d'ammonium, acide borique, acide citrique, acide lactique, acide phosphorique, chlorure de potassium, citrate de potassium, métaphosphate de potassium, phosphate (monobasique) de potassium, acétate de sodium, citrate de sodium, lactate de sodium phosphate (dibasique) de sodium, phosphate (monobasique) de sodium et mélanges de ceux-ci.

9. Méthode selon l'une quelconque des revendications 1 à 8, où ledit tampon salin est un tampon phosphate salin (PBS), éventuellement où ledit PBS comprend du chlorure de sodium, du chlorure de potassium, du phosphate de sodium et du phosphate de potassium.

10. Méthode selon l'une quelconque des revendications 1 à 9, où ladite étape de fixation j) est effectuée en immergeant ledit substrat dans un agent de fixation sélectionné parmi les suivants : paraformaldéhyde, formaldéhyde, glutaraldéhyde, oxyde de mercure, oxyde de plomb, oxyde d'osmium, acide trichloroacétique, acide acétique et mélanges de ceux-ci, préférablement où ledit agent de fixation est le paraformaldéhyde.

11. Méthode selon l'une quelconque des revendications 1 à 10, où ladite étape de lavage k) est effectuée dans une solution aqueuse.

12. Biomatériau fibreux à base de fibrogène produit par une méthode selon l'une quelconque des revendications 1 à 11.

13. Biomatériau fibreux à base de fibrogène selon la revendication 12 ou composition comprenant ledit biomatériau fibreux à base de fibrogène pour une utilisation en tant que médicament.

14. Biomatériau fibreux à base de fibrogène selon la revendication 12 ou composition comprenant ledit biomatériau fibreux à base de fibrogène pour une utilisation dans le traitement d'une maladie dégénérative, d'une plaie, d'une maladie dermatologique, d'une lésion vasculaire et/ou d'une lésion cutanée.
